# EUROPÄISCHE PATENTSCHRIFT

(11) **EP 1 232 134 B1**
(45) Veröffentlichungstag und Bekanntmachung des Hinweises auf die Patenterteilung: **22.09.2004**
(21) Anmeldenummer: 00971412.2
(22) Anmeldetag: 03.11.2000
(51) Int. Cl.: C07C 39/16, C07C 37/68, C07C 37/20

(54) **VERFAHREN ZUR HERSTELLUNG VON BISPHENOL-A**
METHOD FOR THE PRODUCTION OF BISPHENOL-A
PROCEDE DE PREPARATION DE BISPHENOL-A

(30) Priorität: 15.11.1999 DE 19954786
(43) Veröffentlichungstag der Anmeldung: 21.08.2002
(73) Patentinhaber: Bayer MaterialScience AG, 51368 Leverkusen (DE)
(72) Erfinder: HEYDENREICH, Frieder, 40593 Düsseldorf (DE); PREIN, Michael, B-2930 Brasschaat (BE); BÖDIGER, Michael, League City, TX 77573 (US); NEUMANN, Rainer, 47803 Krefeld (DE)
(86) Internationale Anmeldenummer: PCT/EP2000/010827
(87) Internationale Veröffentlichungsnummer: WO 2001/036358

(56) Entgegenhaltungen:
- EP-A- 0 829 464
- US-A- 5 414 151

## Beschreibung

Die Erfindung betrifft ein Verfahren zur effizienten Herstellung von hochreinen Bisphenolen.

Bisphenole sind wichtige Rohstoffe für die Herstellung von Polymeren wie Epoxy-Harze oder insbesondere Polycarbonate. Für diesen Einsatz werden große Anforderungen an die Reinheit der Bisphenole gestellt, so dass für ökonomische, großtechnische Herstellungsverfahren neben der Erzielung hoher Umsätze und Selektivitäten in der Reaktion insbesondere den Aufarbeitungsschritten Bedeutung zukommmt.

Bisphenole werden nach prinzipiell bekannten Verfahren durch die Kondensation von Carbonylverbindungen mit aromatischen Alkoholen in Gegenwart von sauren Katalysatoren hergestellt. Ein Verfahren für die technisch bedeutende Herstellung von 2,2-Bis-(4-hydroxyphenyl)propan (BPA) ist die Umsetzung von Aceton und Phenol in Gegenwart von vernetzten sulfonierten Polystyrolharzen (Ionentauscherharzen). Hierbei wird ein Phenol-/Acetonverhältnis von mindestens 5:1 eingestellt. Zur Erzielung hoher Selektivitäten kommen Cokatalysatoren zum Einsatz, die entweder homogen in den Reaktanden gelöst sind oder durch kovalente oder ionische Bindungen am Ionentauscherharz fixiert sind.

Aufgabe ist, die in dem obigen Verfahren zur Herstellung von BPA entstehenden Nebenprodukte durch geeignete Maßnahmen von BPA abzutrennen und überschüssiges Phenol restlos aus dem Produkt zu entfernen. Außerdem sollten die bei diesen Maßnahmen entstehenden Seitenströme auf ökonomischen Wege im Gesamtprozess wiederverwertet werden.

Als Lösung dieser Aufgaben wird in der Literatur die Isolierung von BPA-Phenol-Adduktkristallen aus der Reaktionslösung durch Suspensionskristallisation mit oder ohne vorherige Destillation zur Entfernung von Wasser, Aceton und Phenol beschrieben (EP-A-829464, EP-A-522700 und EP-A-671377). Hierbei wird die bei der Filtration der Adduktkristalle entstehende stark phenolhaltige Mutterlauge, gegebenenfalls nach Einschaltung einer Umlagerungsreaktion, vor die Reaktionseinheit zurückgeführt und mit frischem Phenol und Aceton ergänzt. Die Suspensionskristallisation kann zur Erzielung höherer Reinheiten gegebenenfalls mehrfach in Serie durchgeführt werden. Nachteile dieses Verfahrens sind die Notwendigkeit zur Nutzung aufwendiger Apparaturen zur Kristallisation und zur Fest-Flüssig-Trennung. Außerdem tritt bei der Suspensionskristallisation die Belegung der Oberflächen der eingesetzten Apparaturen mit BPA oder BPA-Phenol-Addukten als Problem auf, die ein regelmäßiges Reinigen der Oberflächen durch Abschmelzen der Ablagerungen erforderlich macht (EP-A-718267).

Zur Umgehung dieser Problematik wurden in EP-A-758637 Verfahren vorgeschlagen, die auf eine Adduktkristallisation und somit die Erzeugung eines Mutterlaugenkreislaufs verzichten. Hierbei wird BPA durch Aufarbeitung des Reaktionsstroms in einer Kaskade destillativer Reinigungschritte durchgeführt, wobei der Reaktionsstrom ohne Generierung von Rückströmen von Aceton, Wasser, Phenol und Nebenprodukten gereinigt wird. Nachteile dieses Verfahrens sind die thermische Belastung des Produkts durch die hohen Temperaturen bei der Destillation, die hohen Energiekosten der Destillationskaskade und der hohe Verlust an Rohstoffen durch den Verzicht auf Umlagerung und Rückführung.

Die EP-A-785181 beschreibt die Aufarbeitung der Reaktionslösung, ebenfalls unter Verzicht auf eine Adduktkristallisation, durch eine Kombination von Vakuumdestillation zur Entfernung von Aceton, Wasser, Phenol und gegebenenfalls Nebenkomponenten und anschließende Schmelzekristallisation. Auch bei diesem Vorgehen sind durch den Verzicht auf eine Rückführung der bei der Reinigung entstehenden BPA-haltigen Nebenproduktströmen, Einbußen an Rohstoffen hinzunehmen.

Das erfindungsgemäße Verfahren zur Aufarbeitung von hochreinem BPA umgeht die oben beschriebenen Nachteile, indem die Adduktkristallisation aus dem Reaktionsstrom in Form einer Schichtkristallisation durchgeführt wird.

Gegenstand der Erfindung ist ein Verfahren zur Herstellung von 2,2-Bis-(4-hydroxyphenyl)propan (BPA) durch Umsetzung von Aceton und Phenol in Gegenwart von vernetzten sulfonierten Polystyrolharzen (Ionentauscherharzen), dadurch gekennzeichnet, dass im Anschluss an die Reaktionseinheit, BPA aus der Reaktionslösung
a) durch eine Primärkristallisation in Form einer kontinuierlich oder diskontinuierlich betriebenen Schichtkristallisation abgetrennt,
b) und durch Destillation oder Kristallisation gereinigt,
c) und der an Nebenprodukten angereicherte von BPA getrennte Kreislaufstrom aus der Schichtkristallisation unter Zwischenschaltung einer Umlagerungsreaktion und einer Destillation zur Entfernung von Wasser, Aceton und gegebenenfalls Phenol vor oder hinter den Reaktor zurückgeführt wird.

Durch diese Verfahrensführung kann auf den Einsatz einer Suspensionskristallisation und den Einsatz von Filtern und/oder Zentrifugen zur mechanischen Fest-Flüssig-Trennung verzichtet werden.

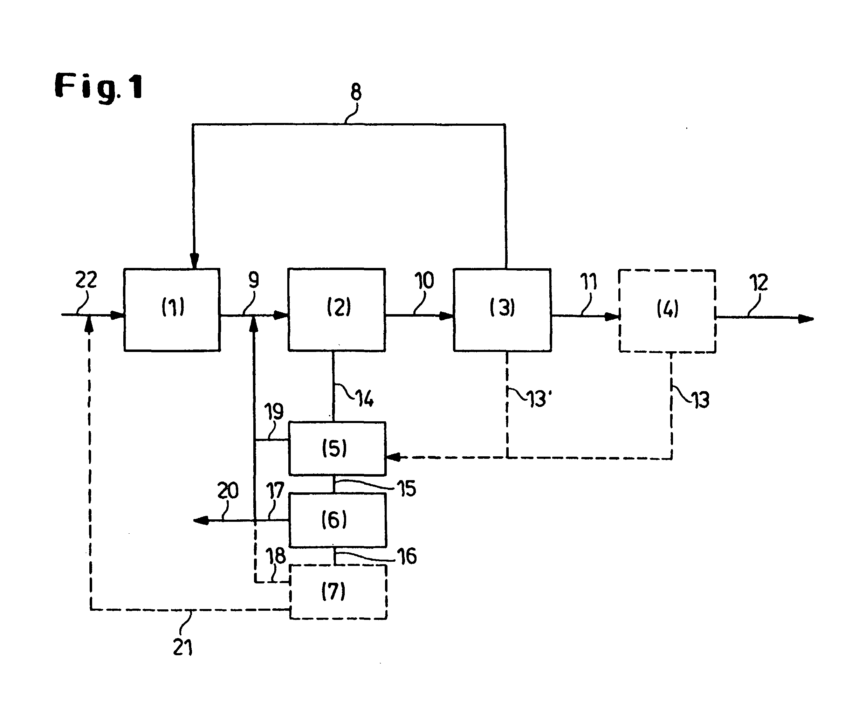

Das erfindungsgemäßen Verfahren wird in dem Verfahrensschema Abb. 1 näher erläutert.
(1) stellt eine Reaktionseinheit dar, in der Phenol, Aceton und gegebenenfalls über den Mutterlaugenkreislauf zurückgeführte Nebenprodukte der BPA-Herstellung mit einem Phenol/Acetonverhältnis von mindestens 5:1, bevorzugt mindestens 10:1 bei Temperaturen von 40 bis 110°C, bevorzugt 45 bis 70°C, einem Ionenaustauscher-Katalysatorsystem, bestehend aus einem sulfonierten vemetzten Polystyrol, zugeführt werden.

Das Ionentauscher-Katalysatorsystem ist entweder mit einer kovalent oder ionisch gebundenen Mercaptoverbindung modifiziert oder eine geeignete Mercaptoverbindung wird homogen der Reaktionslösung zudosiert und im Kreis geführt.

Die Reaktionseinheit ist beispielhaft und vorzugsweise ein Schichtbett oder Wirbelbett, die auf- oder abwärts durchflossen werden, oder eine Kolonne nach Art einer Reaktivdestillation.

Der aus der Reaktionseinheit (1) austretende Reaktionsstrom (Reaktionslösung) enthält nicht umgesetztes Phenol und Aceton, daneben Wasser, BPA und die bei der Reaktion typischerweise entstehenden Nebenkomponenten wie o,p-BPA, Indane, Chromane, und höher kondensierte Reaktionsprodukte mit drei oder mehr aromatischen Kernen.

Dieser Reaktionsstrom wird einer Kristallisationseinheit (2) zugeführt in der die Kristallisation als Schichtkristallisation an gekühlten Oberflächen in einer kontinuierlich oder diskontinuierlich betriebenen Schmelzekristallisationsapparatur bei Temperaturen von 30 bis 110°C, bevorzugt 35 bis 80°C erfolgt. Die Kristallisation kann statisch oder in Form einer Fallfilmkristallisation durchgeführt werden.

Nach Beendigung des Kristallwachstums werden die gebildeten Kristalle durch Ablassen der flüssigen Mutterlauge isoliert. Gegebenenfalls wird anschließend eine weitere Reinigung der Kristalle, beispielhaft und vorzugsweise mittels Durchlaufen eines Temperaturgradienten durch Schwitzen, durchgeführt. Abschließend werden die Kristalle durch Erhöhung der Temperatur über den Schmelzpunkt verflüssigt und zur weiteren Verarbeitung in einen Sammelbehälter abgelassen. Die hierbei erhaltene Mischung enthält p,p-BPA (50-70 %), Phenol (30-50 %) und Nebenkomponenten (0,1-10 %).

Diese Mischung wird einer Einheit zur Phenolentfernung (3) zugeführt, in der Phenol nach prinzipiell bekanntem Verfahren, beispielhaft und vorzugsweise durch Destillation oder Desorption, bis zu Gehalten von <0,5 %, bevorzugt <0,1 % entfernt wird.

Gegebenenfalls wird das so erhaltene p,p-BPA anschließend in einer zweiten Reinigungseinheit (4) beispielhaft und vorzugsweise entweder durch ein- oder mehrstufige Destillation oder erneute Schichtkristallisation auf eine Reinheit von mindestens 99,5 %, bevorzugt mindestens 99,85 % aufgereinigt.

Der in der Kristallisationeinheit (2) erhaltenen Mutterlaugenstrom wird bevorzugt über einen Umlagerungsreaktor (5) geführt, der mit saurem Ionentauscher befüllt ist und bei Temperaturen von 50 bis 110°C, bevorzugt 60 bis 80°C betrieben wird. Hierdurch wird die Umlagerung eines Teils der im Rückstrom enthaltenen Nebenprodukte (o,p-BPA, höhere Kondensate) in p,p-BPA bewirkt und so die Produktausbeute erhöht.

Im Umlagerungsreaktor (5) werden bei Bedarf gegebenenfalls Phenol und/oder Aceton zudosiert.

Der am Ablauf des Umlagerungsreaktor (5) erhaltenen Stoffstrom wird einer mehrstufigen destillativen Aufarbeitung zugeführt, wobei in einer Destillationseinheit (6) Wasser und Aceton und optional in einer Destillationseinheit (7) Phenol abgetrennt werden.

Das hierbei erhaltene Phenol wird zur Reaktionseinheit (1) zurückgeführt und für die Herstellung von BPA genutzt.

Der an BPA und Isomeren angereicherte Sumpfstrom der Destillationseinheit (7) wird vor die Kristallisationseinheit (2) geführt.

In einer alternativen Verfahrensvariante wird auf die Abtrennung von Phenol in der Destillationseinheit (7) verzichtet und der Mutterlaugenstrom direkt, nach Abtrennung von Wasser und Aceton in der Destillationseinheit (6), zur Reaktionseinheit (1) rückgeführt. Ein Teil des Mutterlaugenstroms kann gegebenenfalls aus dem Prozeß abgeführt werden.

Im Verfahrensschema Abb. 1 steht desweiteren
(8) für eine Phenol-Rückführleitung
(9) bis (11) für Reaktionsstromleitungen
(12) für eine Produktentnahmeeinheit
(13) und (13) für eine Nebenproduktstromleitung
(14) bis (19) für Mutterlaugenstrom-Leitungen
(20) für eine Entnahmeeinheit
(21) für eine Phenol-Rückführleitung
(22) für eine Zuführungseinheit für Phenol und Aceton

Durch das erfindungsgemäße Verfahren wird qualitativ hochwertiges BPA in ökonomischer Weise unter Minimierung von Rückströmen, Energieeintrag und Stoffverlusten erhalten. Bei der Herstellung kann auf den Einsatz von Drehfiltern und Zentrifugen zur Abtrennung von Adduktkristallen bei der Suspensionskristallisation von BPA verzichtet werden. Somit wird der technisch aufwendige, wartungsintensive Betrieb von Prozessapparaturen vermieden und die Verfügbarkeit der Anlage erhöht. Das auf diesem Wege hergestellte BPA ist zur Nutzung als Rohstoff für Polymere wie Polycarbonate oder Epoxy-Harze aufgrund seiner hohen Reinheit und guten Farbzahl besonders geeignet.

Die nachfolgenden Beispiele dienen zur Erläuterung der Erfindung. Die Erfindung ist nicht auf die Beispiele limitiert. Prozentangaben bedeuten nachfolgend Gewichtsprozente.

### Beispiele

### Beispiel 1

Es wird eine kontinuierlich arbeitende Versuchsapparatur nach Abb. 1 mit Phenolabtrennung (7) betrieben. Dabei werden im Gleichgewicht in den einzelnen Verfahrensstufen folgende Bedingungen und Konzentrationen eingestellt:

**Reaktionseinheit (1):** Befüllt mit phenolfeuchtem sulfoniertem Polystyrolharz (Lewatit SC 104, Bayer AG, modifiziert mit 5 % Cysteamin), Eingangstemperatur: 65°C, Durchsatz: 0,21 Reaktionslösung/ 1 Katalysator * h
Konzentration am Eingang: Phenol 96 %, Aceton 4,0 %
Konzentration am Ausgang: Phenol 84,5 %, Aceton 0,25 %, Wasser 1,45 %, p,p-BPA 12,8 %, Nebenkomponenten 1,0 %.

**Schichtkristallisation (2):** Betrieben als statische Schichtkristallisation mit folgenden Einzelschritten: Kristallisation (63°C bis 36°C), Ablassen der Mutterlauge, Schwitzen (36°C bis 61°C), Abschmelzen des Produkts (61°C bis 110°C).

Konzentration am Eingang: Phenol 70,2 %, Aceton 0,2 %, Wasser 1,4 %, p,p-BPA 21,2 %, Nebenkomponenten 7,0 %.

Konzentration am Ausgang, Produktstrom: Phenol 42,2 %, Aceton <0,1 %, Wasser <0,1 %, p,p-BPA 56,4 %, Nebenkomponenten 1,4 %.

Konzentration am Ausgang, Mutterlaugenstrom: Phenol 85,0 %, Aceton 0,3 %, Wasser 2,2 %, p,p-BPA 2,5 %, Nebenkomponenten 10,0 %.

**Phenolentfernung (3):** Betrieben als einstufige Desorptionseinheit mit Stickstoff, Sumpftemperatur 190°C

Konzentration am Eingang: Phenol 42,2 %, p,p-BPA 56,4 %, Nebenkomponenten 1,4 %.

Konzentration am Ausgang: Phenol <0,1 %, p,p-BPA 97,6 %, Nebenkomponenten 2,4%.

**Endreinigung (4):** Betrieben als statische Schichtkristallisation mit folgenden Einzelschritten: Kristallisation, Ablassen der Mutterlauge, Schwitzen, Abschmelzen des Produkts.

Konzentration am Eingang: Phenol <0,1 %, p,p-BPA 97,6 %, Nebenkomponenten 2,4%.

Konzentration am Ausgang: p,p-BPA 99,84 %, Nebenkomponenten 0,16 %

**Umlagerungsreaktion (5):** Befüllt mit phenolfeuchtem sulfoniertem Polystyrolharz (Lewatit SC 104, Bayer AG, modifiziert mit 5 % Cysteamin), Eingangstemperatur: 80°C, Durchsatz: 0,21 Reaktionslösung/ 1 Katalysator * h

**Wasser-/Acetonentfernung (6):** Packungskolonne betrieben bei 150 mbar, 140°C Sumpftemperatur, Abtrennung von Wasser und Aceton im Produktstrom auf <0,1 %

**Phenolabtrennung (7):** Packungskolonne betrieben bei 100 mbar, Kopftemperatur 120°C, der vor die Reaktionseinheit zurückgeführte Phenolstrom zeigte eine Reinheit von 99,9 %.

Die Menge an ausgeschleustem BPA-Harz aus dem Rückstrom vor die Kristallisationseinheit betrug 8 % der rückgeführten Menge.

## Patentansprüche

1. Gegenstand der Erfindung ist ein Verfahren zur Herstellung von p,p-Bisphenol A (BPA) durch Umsetzung von Aceton und Phenol in Gegenwart von vernetzten sulfonierten Polystyrolharzen (Ionentauscherharzen), **dadurch gekennzeichnet, dass** im Anschluss an die Reaktionseinheit, BPA aus der Reaktionslösung
a) durch eine Primärkristallisation in Form einer kontinuierlich oder diskontinuierlich betriebenen Schichtkristallisation,
b) und gegebenenfalls durch eine weitere Reinigung durch Destillation oder Kristallisation abgetrennt,
c) und der an Nebenprodukten angereicherte von BPA getrennte Kreislaufstrom unter Zwischenschaltung einer Umlagerungsreaktion und einer Destillation zur Entfernung von Wasser, Aceton und gegebenenfalls Phenol vor oder hinter den Reaktor zurückgeführt wird.

2. Verfahren nach Anspruch 1, **dadurch gekennzeichnet, dass** das zur Herstellung verwendete vernetzte sulfonierte Polystyrolharz mit einer kovalent oder ionisch gebundenen Mercaptoverbindung modifiziert ist.

3. Verfahren nach Anspruch 2, **dadurch gekennzeichnet, dass** die ionisch gebundene Mercaptoverbindung 2-Mercaptoethylamin (Cysteamin) ist.

## Claims

1. The invention provides a process for the production of p,p-bisphenol A (BPA) by reacting acetone and phenol in the presence of cross-linked sulfonated polystyrene resins (ion-exchange resins), **characterised in that**, subsequent to the reaction unit, BPA is separated off from the reaction solution
a) by a primary crystallisation in the form of a layer crystallisation operated continuously or batchwise,
b) and optionally by a further purification by distillation or crystallisation,
c) and the circulating flow of material, separated from BPA and enriched with secondary products, with the insertion of a rearrangement reaction and of a distillation for the removal of water, acetone and optionally phenol, is returned to the front or the rear of the reactor.

2. Process according to claim 1, **characterised in that** the cross-linked sulfonated polystyrene resin used for the production is modified with a covalently or ionically bonded mercapto compound.

3. Process according to claim 2, **characterised in that** the ionically bonded mercapto compound is 2-mercaptoethylamine (cysteamine).

## Revendications

1. L'objet de l'invention est un procédé de préparation de p,p-Bisphénol A (BPA) par conversion d'acétone et de phénol en présence de résines de polystyrène sulfonées réticulées (résines échangeuses d'ions), **caractérisé en ce que**, à la suite de l'unité de réaction,
a) on sépare le BPA hors de la solution de réaction par une cristallisation primaire sous la forme d'une cristallisation en couches menée de façon continue ou discontinue,
b) on le sépare éventuellement par une autre purification par distillation ou cristallisation,
c) et on renvoie, avant ou après le réacteur, le courant de circulation, séparé du BPA et enrichi en sous-produits, en intercalant une réaction de regroupement et une distillation pour éliminer l'eau, l'acétone et éventuellement le phénol.

2. Procédé selon la revendication 1, **caractérisé en ce que** la résine de polystyrène sulfonée réticulée utilisée pour la préparation est modifiée avec un composé de mercaptan à liaison covalente ou ionique.

3. Procédé selon la revendication 2, **caractérisé en ce que** le composé de mercaptan à liaison ionique est la 2-mercapto-éthylamine (amine cystéique).
